# EUROPÄISCHE PATENTSCHRIFT

(11) **EP 0 817 609 B1**
(45) Veröffentlichungstag und Bekanntmachung des Hinweises auf die Patenterteilung: **26.06.2002**
(21) Anmeldenummer: 96908085.2
(22) Anmeldetag: 20.03.1996
(51) Int. Cl.: A61K 7/50, C11D 1/66, C11D 1/86, C11D 1/825

(54) **PERLGLANZKONZENTRAT MIT NEWTON'SCHEM VISKOSITÄTSVERHALTEN**
PEARLY LUSTRE CONCENTRATE WITH NEWTONIAN VISCOSITY
CONCENTRE D'ASPECT NACRE A VISCOSITE NEWTONIENNE

(30) Priorität: 29.03.1995 DE 19511571
(43) Veröffentlichungstag der Anmeldung: 14.01.1998
(73) Patentinhaber: Cognis Deutschland GmbH & Co. KG, 40589 Düsseldorf (DE)
(72) Erfinder: ANSMANN, Achim, D-40699 Erkrath (DE); KAWA, Rolf, D-40789 Monheim (DE); STRAUSS, Gabriele, D-40589 Düsseldorf (DE)
(86) Internationale Anmeldenummer: EP9601198
(87) Internationale Veröffentlichungsnummer: WO9629981

(56) Entgegenhaltungen:
- EP-A- 0 268 992
- EP-A- 0 300 379
- EP-A- 0 376 083
- WO-A-92/13512
- DE-A- 19 511 574

## Beschreibung

Gegenstand der Erfindung ist ein Perlglanzkonzentrat in Form einer niedrigviskosen wäßrigen Dispersion mit einem Gehalt von 10 - 40 Gew.-% an perlglanzbildenden Komponenten, das newton'sches Strömungsverhalten aufweist.

Wäßrigen Zubereitungen von Tensiden und kosmetischen Präparaten kann man durch Einarbeitung von Substanzen, die nach dem Abkühlen in Form feiner, perlmuttartig aussehender Kristalle ausfallen und in den Zubereitungen dispergiert bleiben, ein perlglänzendes, ästhetisch ansprechendes Aussehen verleihen. Als perlglanzbildende Stoffe eignen sich z. B. die Mono-, Diund gegebenenfalls Triester von Ethylenglykol oder Glycerin mit C₁₄-C₂₂-Fettsäuren, oligomere Alkylenglykolester dieser Art, wie beispielsweise PEG-3-distearate, Fettsäuren sowie Monoalkanolamide von Fettsäuren.

Es ist auch bekannt, die genannten Perlglanzbildner in Wasser oder in wäßrigen Emulgatorlösungen stabil zu dispergieren und die auf diese Weise erhaltenen konzentrierten Perlglanzdispersionen den perlglänzend auszustattenden Zubereitungen ohne Erwärmung zuzusetzen, so daß sich das für die Einarbeitung sonst erforderliche Erwärmen und Abkühlen zur Bildung der Perlglanzkristalle erübrigt.

Ein Problem bei der Herstellung und Anwendung von Perlglanzkonzentraten stellt die Fließ- und Pumpfähigkeit dar. Vor allem bei hohen Konzentrationen an perlglanzbildenden Komponenten und Emulgatoren ist die Fließund Pumpfähigkeit häufig stark eingeschränkt, oder es liegen sogar Mischungen vor, die nicht fließfähig sind und auch mit den gängigen Apparaturen nicht pumpbar sind.

In den deutschen Patentanmeldungen DE 38 43 572 und DE 41 03 551 wurde daher vorgeschlagen, die Viskosität von Perlglanzkonzentraten durch Zugabe von niedermolekularen, mehrwertigen Alkoholen zu senken und dadurch Fließund Pumpfähigkeit zu erreichen.

In der internationalen Patentanmeldung WO 94/24248 wurden Perlglanzmittel auf einer Alkylpolyglykosid/Betain-Tensidbasis vorgeschlagen, die ebenfalls Glykole enthalten.

Wenngleich nach den Lehren dieser Druckschriften Perlglanzkonzentrate bereitgestellt werden können, die hinreichend pump- bzw. fließfähig für eine übliche Verarbeitung sind, können diese Produkte noch nicht vollständig befriedigen. Ursache dafür ist das ausgeprägt nichtnewton'sche oder thixotrope Verhalten dieser Konzentrate.

So wird insbesondere nach längeren Lagerungszeiten ein thixotroper Viskositätsaufbau beobachtet. Weiterhin bleibt aufgrund der Fließgrenze im Vorratsbehälter stets eine mehr oder weniger dicke Produktschicht an den Wandungen zurück, die nur mit immensem Aufwand oder im Rahmen von Reinigungsoperationen entfernt werden kann. Im letzteren Fall geht somit ein Teil des Produktes für die Verarbeitung verloren. Schließlich ist der Druckverlust beim Pumpen für nicht-newton'sche Flüssigkeiten deutlich höher als für newton'sche Flüssigkeiten, was insbesondere zu Beginn des Pumpvorganges erhöhte Leistungsanforderungen an die Pumpeinrichtung stellt.

Es besteht daher nach wie vor ein Bedarf an Perlglanzkonzentraten, die sich sowohl durch niedrige Viskositäten, d.h. Pump- oder Fließfähigkeit, insbesondere aber auch durch ein newtonsches Viskositätsverhalten auszeichnen.

Es wurde nun überraschenderweise gefunden, daß bei Verwendung von Alkylpolyglykosiden als Emulgatoren niedrigviskose Perlglanzkonzentrate formuliert werden können, die das Viskositätsverhalten newtonscher Flüssigkeiten aufweisen. Dieses Viskositätsverhalten tritt nur auf, wenn die eingesetzten Emulgatoren überwiegend zur Klasse der Alkylpolyglykoside gehören; z.B. führen in der Regel bereits geringe Mengen an Emulgatoren mit -COO⁻- und -OSO₃⁻-Gruppen zu ausgeprägt nichtnewton'schem Verhalten der Perlglanzkonzentrate.

Gegenstand der Anmeldung ist somit ein Perlglanzkonzentrat in Form einer wäßrigen Dispersion mit 10 - 40 Gew.-% an perlglanzbildenden Komponenten und 15 - 55 Gew.-% an Emulgatoren, dadurch gekennzeichnet, daß es sich bei den Emulgatoren um Alkylpolyglykoside der allgemeinen Formel (I),

RO-(Z)ₓ (I),

in der R steht für einen Alkylrest mit 6 bis 22 Kohlenstoffatomen, Z für einen Mono- oder Oligosaccharid, x für eine Zahl von 1,1 bis 5, oder deren Anlagerungsprodukte mit 1 bis 10 Molekülen Ethylenoxid und/oder Propylenoxid handelt, und das Perlglanzkonzentrat frei von Emulgatoren mit -COO⁻- und -OSO₃⁻-Gruppen ist.

Unter perlglanzbildenden Komponenten werden schmelzbare Fett- oder Wachskörper verstanden, die beim Abkühlen ihrer wäßrigen Lösungen oder Emulsionen in einem Temperaturbereich von etwa 30 - 90 °C in Form feiner, perlglänzender Körper auskristallisieren.

Zu diesen schmelzbaren Fett- oder Wachskörpern gehören
(A1) Ester der Formel (II),

   R¹ - (OCₙH₂ₙ)ₓ - OR² (II),

   in der R¹ eine lineare Fettacylgruppe mit 14 bis 22 C-Atomen, R² Wasserstoff oder eine Gruppe R¹, n = 2 oder 3 und x eine Zahl von 1 bis 4 ist,
(A2) Monoalkanolamide der allgemeinen Formel (III),

   R³ - CO -NH - X (III),

   in der R³ eine Alkylgruppe mit 8 bis 22 C-Atomen, insbesondere mit 8 bis 18 C-Atomen, und X eine Gruppe -CH₂-CH₂-OH, eine Gruppe -CH₂-CH₂-CH₂-OH oder eine Gruppe -C(CH₃)₂-OH darstellt,
(A3) lineare, gesättigte Fettsäuren mit 14 bis 22 C-Atomen,
(A4) Mono-, Di- und Triester des Glycerins mit linearen, gesättigten Fettsäuren mit 12 bis 22 C-Atomen sowie
(A5) β-Ketosulfone der allgemeinen Formel (IV), 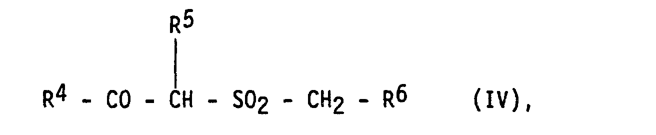 in der R⁴ eine Alkyl- oder Alkenylgruppe mit 11 bis 21 C-Atomen, R⁵ und R⁶ Wasserstoffatome oder gemeinsam eine Ethylengruppe, die mit der zwischen R⁵ und R⁶ liegenden Gruppe einen Tetrahydrothiophendioxidring bildet, darstellen.

Als Ester (A1) der allgemeinen Formel R¹(OCₙH₂ₙ)ₓOR² können z. B. die Mono- und Diester des Ethylenglykols und Propylenglykols mit höheren Fettsäuren, z. B. mit Palmitinsäure, Stearinsäure oder Behensäure, oder die Diester des Diethylenglykols oder des Triethylenglykols mit solchen Fettsäuren eingesetzt werden. Geeignet sind auch Mischungen von Mono- und Diestern der genannten Glykole mit Fettsäuregemischen, z. B. mit gehärteter Talgfettsäure, Palmfettsäure oder mit der gesättigten C₁₄-C₁₈-Fettsäurefraktion der Talgfettsäure. Bevorzugt geeignet sind der Ethylenglykolmonound/oder -diester der Palmitin- und/oder Stearinsäure.

Bevorzugte Monoalkanolamide (A2) sind die Monoethanolamide. Diese Verbindungen können einen einheitlichen Alkylrest enthalten. Es ist jedoch üblich, bei der Herstellung der Alkanolamide von Fettsäuregemischen aus natürlichen Quellen, z.B. Kokosfettsäuren, auszugehen, so daß entsprechende Mischungen bezüglich der Alkylreste vorliegen.

Als lineare Fettsäuren (A3) können z. B. Palmitinsäure, Stearinsäure, Arachinsäure oder Behensäure eingesetzt werden, geeignet sind aber auch technische Fettsäureschnitte, die ganz oder überwiegend aus Fettsäuren mit 16 bis 22 C-Atomen bestehen, z. B. Palmitin-Stearinsäure-Fraktionen, wie sie aus Talgfettsäure oder Palmfettsäure durch Abtrennung der bei +5 °C flüssigen Fettsäuren gewonnen werden oder Palmitin-Stearinsäure-Fraktionen, wie sie durch Härten von Talgfettsäure oder Palmfettsäure erhältlich sind.

Zu den in der erfindungsgemäßen Lehre verwendbaren Estern des Glycerins (A4) gehören die Mono-, Di- und insbesondere Triester mit Laurinsäure, Myristinsäure, Palmitinsäure, Stearinsäure und Behensäure, sowie mit Mischungen dieser Fettsäuren.

Gegenüber den bekannten Ethylenglykolmono- und -diestern haben die β-Ketosulfone (A5) der allgemeinen Formel (IV) den Vorteil, daß der Perlglanz der Zubereitungen eine höhere Thermostabilität aufweist, d.h. daß der Perlglanz beim Erwärmen der Zubereitungen auf über 50 °C, teilweise sogar auf über 70 °C, über mehrere Stunden erhalten bleibt. Hinsichtlich weiterer Informationen zu den genannten β-Ketosulfonen wird ausdrücklich auf den Inhalt der deutschen Patentanmeldung 35 08 051 hingewiesen.

Die erfindungsgemäßen Perlglanzkonzentrate können sowohl ausschließlich Vertreter einer dieser Verbindungsklassen als auch Mischungen von Vertretern mehrerer dieser Verbindungsklassen enthalten.

Bevorzugte perlglanzbildende Komponenten sind Vertreter der Klassen (A1) bis (A4).

Fettsäure-mono- oder -dialkanolamide, also perlglanzbildende Komponenten der Gruppe (A2), und deren Derivate werden aber in jüngster Zeit verdächtigt, an der Bildung von Nitroaminen beteiligt zu sein. Es kann daher erwünscht sein, kosmetische Zubereitungen ohne solche Alkanolamine und Alkanolamin-Derivate zu formulieren. Aus diesem Grunde können Verbindungen der Klassen (A1), (A3) und (A4) besonders bevorzugte perlglanzbildende Komponenten sein.

Ganz besonders bevorzugt sind solche Perlglanzkonzentrate, deren perlglanzbildende Komponenten zu mindestens 50 Gew.-%, insbesondere zu mindestens 70 Gew.-%, aus Ethylenglykoldistearat bestehen.

Als Emulgatoren enthalten die erfindungsgemäßen Perlglanzkonzentrate ausschließlich oder überwiegend Alkylpolyglykoside.

Die Alkylpolyglykoside gemäß Formel (I) sind durch folgende Parameter gekennzeichnet:

Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylglykoside mit x-Werten von 1,1 bis 2 sind bevorzugt. Ganz besonders bevorzugt sind Alkylglykoside, bei denen x 1,1 bis 1,4 beträgt.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten. Auch diese Produkte stellen üblicherweise keine einheitlichen Verbindungen dar, sondern weisen in Abhängigkeit von dem gewählten Ethoxylierungsverfahren eine entsprechende Homologenverteilung auf. Solche alkoxylierten Verbindungen können beispielsweise dadurch erhalten werden, daß zur Synthese der Alkylpolyglykoside ethoxylierte Fettalkohole verwendet werden. Im Rahmen der erfindungsgemäßen Lehre ist die Verwendung der nicht alkoxylierten Verbindungen allerdings bevorzugt.

Wie bereits aufgeführt, ist das newton'sche Viskositätsverhalten der Perlglanzkonzentrate auf die Wahl der Alkylpolyglykoside als Emulgatoren zurückzuführen. Es wurde jedoch auch gefunden, daß in einer Reihe von Fällen die Zugabe weiterer Emulgatoren nicht zum Verlust dieses Viskositätsverhaltens führt. Bei diesen Emulgatoren handelt es sich insbesondere um weitere nichtionische Emulgatoren. Diese können in vielen Fällen in Mengen bis zu 50 Gew.-%, insbesondere in Mengen bis 20 Gew.-%, bezogen auf die Mengen an Alkylpolyglykosid, enthalten sein, ohne nichtnewton'sches Viskositätsverhalten zu induzieren.

Geeignete nichtionogene Emulgatoren enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- Sorbitanmono- und -diester von gesättigten und ungesättigten C₈-C₂₂-Fettsäuren und deren Ethylenoxidanlagerungsprodukte und
- Anlagerungsprodukte von 5 bis 60 Mol Ethylenoxid an Ricinusöl und gehärtetes Ricinusöl.

Ebenfalls geeignet sind Mischungen von Verbindungen aus mehreren dieser Substanzklassen.

Weiterhin haben sich in einer Reihe von Fällen auch kationische Emulgatoren als erfindungsgemäß geeignete, weitere Emulgatoren erwiesen.

Schließlich können erfindungsgemäß in der Regel auch anionische Emulgatoren eingesetzt werden, die als ionische Gruppe eine Phosphatgruppe aufweisen.

Ist beabsichtigt, neben den Alkylpolyglykosiden noch weitere Emulgatoren einzusetzen, so ist der Fachmann im Rahmen der hier gegebenen Lehre in der Lage, die jeweiligen Systeme routinemäßig anhand der ihm bekannten Verfahren zur Viskositätsmessung auf newton'sches Viskositätsverhalten zu überprüfen.

Bei den als Emulgatoren eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen und tierischen Rohstoffen auszugehen, so daß man Substanzgemische mit unterschiedlichen, vom jeweiligen Rohstoff abhängigen Alkylkettenlängen erhält.

Bei den Emulgatoren, die Anlagerungsprodukte von Ethylen- und/oder Propylenoxid an Fettalkohole darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Perlglanzkonzentrate, die 20-30 Gew.-% an perlglanzbildenden Komponenten und 15-45 Gew.-% an Emulgatoren enthalten, haben sich als erfindungsgemäß besonders vorteilhaft erwiesen.

Weiterhin können die erfindungsgemäßen Perlglanzkonzentrate gewünschtenfalls einen niedermolekularen, mehrwertigen Alkohol enthalten.

Eine Gruppe bevorzugt eingesetzter niedermolekularer, mehrwertiger Alkohole enthalten 2-12 Kohlenstoffatome und 2-10 Hydroxylgruppen. Solche Alkohole sind beispielsweise Ethylenglykol, 1,2- und 1,3-Propylenglykol, Glycerin, Erythrit, Arabit, Adonit, Xylit, Sorbit, Mannit, Dulcit, Glucose und Saccharose. Die Verwendung von Glycerin, 1,2-Propylenglykol, 1,3-Propylenglykol, Sorbit und/oder Glucose ist besonders bevorzugt.

Die Verwendung von Glycerin als niedermolekularer, mehrwertiger Alkohol führt zu Perlglanzkonzentraten, die den Endprodukten einen besonders brillanten Perlglanz verleihen.

Eine weitere Gruppe bevorzugt eingesetzter niedermolekularer, mehrwertiger Alkohole stellen oligomere Ether, insbesondere auf Basis von Ethylenglykol, Propylenglykol und Glycerin dar. Dabei sind vor allem solche Produkte geeignet, deren mittlere Molmasse unterhalb von etwa 700 Dalton liegt. Vor allem die Di-, Tri- und Tetrameren von Ethylenglykol und Glycerin sind erfindungsgemäß verwendbar.

Die niedermolekularen, mehrwertigen Alkohole werden erfindungsgemäß bevorzugt in Mengen von 0,1 bis 15 Gew.-%, bezogen auf die gesamte Zubereitung, eingesetzt.

Daneben enthalten die erfindungsgemäßen Perlglanzkonzentrate im wesentlichen Wasser.

Weiterhin können in untergeordneten Mengen Puffersubstanzen zur Einstellung des pH-Wertes auf Werte zwischen 2 und 8, z. B. Citronensäure und/oder Natriumcitrat, sowie anorganische Salze, beispielsweise Natriumchlorid, als Verdickungsmittel enthalten sein.

In einer ersten, bevorzugten Ausführungsform sind die erfindungsgemäßen Perlglanzkonzentrate mit den üblichen, dem Fachmann bekannten Konservierungsmitteln versehen. Solche Konservierungsmittel sind beispielsweise Ameisensäure, Benzoesäure und pHB-Ester.

In einer zweiten bevorzugten Ausführungsform sind die Perlglanzkonzentrate konservierungsmittelfrei. Unter konservierungsmittelfrei werden in diesem Zusammenhang Perlglanzkonzentrate verstanden, denen keine Konservierungsmittel zugesetzt wurden. Sie enthalten daher bevorzugt keine Konservierungsmittel bzw. Konservierungsmittel nur in solchen Mengen, wie sie aufgrund der gewählten einzelnen Rohstoffe mit diesen eingebracht werden.

Die erfindungsgemäßen Perlglanzkonzentrate sind mindestens in einem Temperaturbereich von 15 - 30 °C sehr gut pumpbar und über einen längeren Zeitraum , d. h. mindestens etwa 6 Monate, lagerstabil.

Die Herstellung der erfindungsgemäßen Perlglanzkonzentrate kann so erfolgen, daß die Komponenten (A), (B) und (C) zunächst gemeinsam auf eine Temperatur erwärmt werden, die etwa 1 bis 30°C oberhalb des Schmelzpunktes liegt. Dies wird in den meisten Fällen eine Temperatur von etwa 60 bis 90°C sein. Sodann wird zu dieser Mischung das auf etwa die gleiche Temperatur erwärmte Wasser hinzugegeben. Falls als Emulgator ein ionischer, wasserlöslicher Emulgator eingesetzt wird, kann es bevorzugt sein, diesen in der Wasserphase aufzulösen und mit dem Wasser zusammen in die Mischung einzubringen. Die wäßrige Phase kann auch bereits gegebenenfalls die Puffersubstanzen gelöst enthalten. Die entstehende Dispersion wird dann unter stetigem Rühren auf Raumtemperatur, d. h. auf etwa 25°C, abgekühlt. Die Viskosität des Perlglanzkonzentrates ist in den allermeisten Fällen so niedrig, daß auf den Einsatz besonderer Rühraggregate wie Homogenisatoren oder andere hochtourige Mischvorrichtungen verzichtet werden kann.

Die erfindungsgemäßen Perlglanzkonzentrate eignen sich zur Herstellung getrübter und perlglänzender flüssiger, wäßriger Zubereitungen wasserlöslicher oberflächenaktiver Stoffe. Sie können z.B. in flüssige Wasch- und Reinigungsmittel wie Spülmittel, flüssige Feinwaschmittel und flüssige Seifen, bevorzugt aber in flüssige Körperreinigungs- und Pflegemittel wie z.B. Haarwaschmittel (Shampoos), flüssige Hand- und Körperwaschmittel, Duschbadzubereitungen, Badezusätze (Schaumbäder), Haarspülmittel oder Haarfärbezubereitungen eingearbeitet werden.

Zur Erzeugung von Perlglanz werden den klaren wäßrigen Zubereitungen bei 0 bis 40 °C die erfindungsgemäßen Perlglanzkonzentrate in einer Menge von 1 bis 10 Gew.-%, insbesondere 1,5 bis 5 Gew.-%, der Zubereitung zugesetzt und unter Rühren darin verteilt. Je nach Zubereitung und Einsatzkonzentration entsteht ein metallisch glänzender, dichter bis schwach glänzender, extrem dichter Perlglanz.

Die folgenden Beispiele sollen den Erfindungsgegenstand näher erläutern, ohne ihn darauf zu begrenzen.

### Beispiele

### 1. Perlglanzkonzentrate

Es wurden Perlglanzkonzentrate mit den in Tabelle 1 aufgeführten Zusammensetzungen hergestellt.

**Tabelle 1:**

| Fließfähiae Perlglanzkonzentrate | | | | | |
|---|---|---|---|---|---|
| | Mischung Nr. Gehalt [Gew.-%] | | | | |
| Komponenten | 1 | 2 | 3 | 5 | 6 |
| Perlglanzbildner: | | | | | |
| Cutina(R) AGS¹ | 20 | 20 | 20 | 20 | 15 |
| Comperlan(R) 100³ | - | - | 3 | - | 5 |
| | | | | | |

| Emulgator: | | | | | |
|---|---|---|---|---|---|
| Plantaren^{(R)}H 1200⁵ | 40 | 30 | 40 | 45 | 30 |
| Dehydol^{(R)} LS4⁶ | - | 5 | - | - | - |
| | | | | | |

| Alkohol: | | | | | |
|---|---|---|---|---|---|
| Glycerin⁹ | - | 10 | - - | 15 | - |
| 1,2-Propylenglykol | 7 | - | - | - | - |
| Glucose | - | - | - | - | 13 |
| | | | | | |

| Wasser, | | | | | |
|---|---|---|---|---|---|
| Mittel zur Viskositäts-und pH-Wert-Einstellung | <------- a d 1 0 0 -------> | | | | |

| | | | | | |
|---|---|---|---|---|---|
| ¹ Ethylenglykoldistearat (mind. 90 % Diester; CTFA-Bezeichnung: Glycol Distearate) (HENKEL) | | | | | |
| ³ Kokosfettsäuremonoethanolamid (ca. 95 % Amid; CTFA-Bezeichnung: Cocamide MEA ) (HENKEL) Zusammensetzung der Fettsäure: ca. 56 % Laurinsäure ca. 21 % Myristinsäure ca. 10 % Palmitinsäure ca. 13 % Stear insäure und Ölsäure | | | | | |
| ⁵ C₁₂-C₁₆-Fettalkohol-1,4-glucosid (ca. 50 % Aktivsubstanz in Wasser; CTFA-Bezeichnung: Lauryl Polyglycose) (HENKEL CORP.) | | | | | |
| ⁶ C₁₂-C₁₄-Fettalkohol + 4 Ethylenoxid (HENKEL) | | | | | |
| ⁹ 86 % in Wasser | | | | | |

## Patentansprüche

1. Perlglanzkonzentrat in Form einer wäßrigen Dispersion mit 10 - 40 Gew.-% an perlglanzbildenden Komponenten und 15 - 55 Gew.-% an Emulgatoren, **dadurch gekennzeichnet, daß** es sich bei den Emulgatoren um Alkylpolyglykoside der allgemeinen Formel (I),
RO-(Z)ₓ (I),
in der R steht für einen Alkylrest mit 6 bis 22 Kohlenstoffatomen, Z für einen Mono- oder Oligosaccharid, x für eine Zahl von 1,1 bis 5, oder deren Anlagerungsprodukte mit 1 bis 10 Molekülen Ethylenoxid und/oder Propylenoxid handelt, und das Perlglanzkonzentrat frei von Emulgatoren mit -COO⁻- und -OSO₃⁻-Gruppen ist.

2. Perlglanzkonzentrat nach Anspruch 1, **dadurch gekennzeichnet, daß** in der Formel (I) Z für Glucose und x für eine Zahl zwischen 1,1 und 2, insbesondere für eine Zahl zwischen 1,1 und 1,4 steht.

3. Perlglanzkonzentrat nach einem der Ansprüche 1 oder 2, **dadurch gekennzeichnet, daß** als perlglanzbildende Komponenten
(A1) Ester der Formel (II),
R¹ - (OCₙH₂ₙ)ₓ - OR² (II),
in der R¹ eine lineare Fettacylgruppe mit 14 bis 22 C-Atomen, R² Wasserstoff oder eine Gruppe R¹, n = 2 oder 3 und x eine Zahl von 1 bis 4 ist,
und/oder
(A2) Monoalkanolamide der allgemeinen Formel (III),
R³ - CO -NH - X (III),
in der R³ eine Alkylgruppe mit 8 bis 22 C-Atomen, insbesondere mit 8 bis 18 C-Atomen, und X eine Gruppe -CH₂-CH₂-OH, eine Gruppe -CH₂-CH₂-CH₂-OH oder eine Gruppe -C(CH₃)₂-OH darstellt,
und/oder
(A3) lineare, gesättigte Fettsäuren mit 14 bis 22 C-Atomen
und/oder
(A4) C₁₂₋₂₂-Fettsäuremono-, -di- oder -triglyceride
enthalten sind.

4. Perlglanzkonzentrat nach Anspruch 3, **dadurch gekennzeichnet, daß** die perlglanzbildenden Komponenten zu mindestens 50 Gew.-%, insbesondere zu mindestens 70 Gew.-%, aus Ethylenglykoldistearat bestehen.

5. Perlglanzkonzentrat nach einem der Ansprüche 1 bis 4, **dadurch gekennzeichnet, daß** es 20 - 30 Gew.-% an perlglanzbildenden Komponenten und 15 - 40 Gew.-% an Emulgatoren enthält.

6. Perlglanzkonzentrat nach einem der Ansprüche 1 bis 5, **dadurch gekennzeichnet, daß** es zusätzlich einen niedermolekularen, mehrwertigen Alkohol enthält.

7. Perlglanzkonzentrat nach Anspruch 6, **dadurch gekennzeichnet, daß** der niedermolekulare, mehrwertige Alkohol in Mengen von 0,1 bis 15 Gew.-% enthalten ist.

8. Perlglanzkonzentrat nach einem der Ansprüche 6 oder 7 **dadurch gekennzeichnet, daß** der niedermolekulare, mehrwertige Alkohol 2 bis 12 C-Atome und 2 bis 10 Hydroxylgruppen enthält.

9. Perlglanzkonzentrat nach Anspruch 8, **dadurch gekennzeichnet, daß** der niedermolekulare, mehrwertige Alkohol ausgewählt ist aus der Gruppe, die aus den Substanzen Glycerin, 1,2-Propylenglykol, 1,3-Propylenglykol, Glucose und Sorbit besteht.

10. Perlglanzkonzentrat nach einem der Ansprüche 6 oder 7, **dadurch gekennzeichnet, daß** der niedermolekulare, mehrwertige Alkohol ausgewählt ist aus Polyethylenglykolen, Polypropylenglykolen und Polyglycerinen mit einer durchschnittlichen Molmasse unterhalb von 700 Dalton.

11. Perlglanzkonzentrat nach einem der Ansprüche 1 bis 10, **dadurch gekennzeichnet, daß** es weiterhin nichtionogene Emulgatoren in Mengen von bis zu 50 Gew.-%, bezogen auf die Menge des Alkylpolyglykosids, enthält.

12. Verfahren zur Herstellung von Perlglanzkonzentraten nach einem der Ansprüche 1 bis 11, **dadurch gekennzeichnet, daß** man eine Mischung der Komponenten auf eine Temperatur erwärmt, die 1 bis 30 °C oberhalb des Schmelzpunktes der Mischung liegt, mit der notwendigen Menge Wasser etwa der gleichen Temperatur mischt und anschließend auf Raumtemperatur abkühlt.

13. Verwendung von Perlglanzkonzentraten nach einem der Ansprüche 1 bis 11 in einer Menge von 0,5 bis 10 Gew.-%, insbesondere von 1,5 bis 5 Gew.-%, zur Herstellung getrübter und perlglänzender flüssiger, wäßriger Zubereitungen wasserlöslicher oberflächenaktiver Stoffe.

14. Verfahren zur Herstellung getrübter und perlglänzender flüssiger, wäßriger Zubereitungen wasserlöslicher oberflächenaktiver Stoffe, **dadurch gekennzeichnet, daß** man den klaren wäßrigen Zubereitungen bei 0 bis 40 °C Perlglanzkonzentrate nach einem der Ansprüche 1 bis 11 in einer Menge von 0,5 bis 10 Gew.-%, insbesondere von 1,5 bis 5 Gew.-%, der Zubereitung zusetzt und unter Rühren darin verteilt.

## Claims

1. A pearlescent concentrate in the form of an aqueous dispersion containing 10 to 40% by weight of pearlescing components and 15 to 55% by weight of emulsifiers, **characterized in that** the emulsifiers are alkyl polyglycosides corresponding to general formula (I):
RO-(Z)ₓ (I)
in which R is an alkyl group containing 6 to 22 carbon atoms, Z is a mono- or oligosaccharide, x is a number of 1.1 to 5,
or addition products thereof with 1 to 10 molecules of ethylene oxide and/or propylene oxide and **in that** the pearlescent concentrate is free from emulsifiers containing -COO- and -OSO₃- groups.

2. A pearlescent concentrate as claimed in claim 1, **characterized in that**, in formula (I), Z stands for glucose and x stands for a number of 1.1 to 2 and, more particularly, for a number of 1.1 to 1.4.

3. A pearlescent concentrate as claimed in claim 1 or 2, **characterized in that** the pearlescing components present are
(A1) esters corresponding to formula (II):
R¹ - (OCₙH₂ₙ)ₓ - OR² (II)
in which R¹ is a linear fatty acyl group containing 14 to 22 carbon atoms, R² is hydrogen or has the same meaning as R¹, n = 2 or 3 and x is a number of 1 to 4,
and/or
(A2) monoalkanolamides corresponding to general formula (III):
R³ - CO -NH - X (III)
in which R³ is an alkyl group containing 8 to 22 and, more particularly, 8 to 18 carbon atoms and X is a group -CH₂-CH₂-OH, a group -CH₂-CH₂-CH₂-OH or a group -C(CH₃)₂-OH,
and/or
(A3) linear saturated fatty acids containing 14 to 22 carbon atoms,
and/or
(A4) C₁₂₋₂₂ fatty acid mono-, di- or triglycerides.

4. A pearlescent concentrate as claimed in claim 3, **characterized in that** at least 50% by weight and, more particularly, at least 70% by weight of the pearlescing components consist of ethylene glycol distearate.

5. A pearlescent concentrate as claimed in any of claims 1 to 4, **characterized in that** it contains 20 to 30% by weight of pearlescing components and 15 to 40% by weight of emulsifiers.

6. A pearlescent concentrate as claimed in any of claims 1 to 5, **characterized in that** it additionally contains a low molecular weight polyhydric alcohol.

7. A pearlescent concentrate as claimed in claim 6, **characterized in that** the low molecular weight polyhydric alcohol is present in quantities of 0.1 to 15% by weight.

8. A pearlescent concentrate as claimed in claim 6 or 7, **characterized in that** the low molecular weight polyhydric alcohol contains 2 to 12 carbon atoms and 2 to 10 hydroxyl groups.

9. A pearlescent concentrate as claimed in claim 8, **characterized in that** the low molecular weight polyhydric alcohol is selected from the group consisting of glycerol, 1,2-propylene glycol, 1,3-propylene glycol, glucose and sorbitol.

10. A pearlescent concentrate as claimed in claim 6 or 7, **characterized in that** the low molecular weight polyhydric alcohol is selected from polyethylene glycols, polypropylene glycols and polyglycerols with an average molecular weight below 700 dalton.

11. A pearlescent concentrate as claimed in any of claims 1 to 10, **characterized in that** it additionally contains nonionic emulsifiers in quantities of up to 50% by weight, based on the quantity of alkyl polyglycoside.

12. A process for producing the pearlescent concentrates claimed in any of claims 1 to 11, **characterized in that** a mixture of components (A), (B) and (C) is heated to a temperature 1 to 30°C above the melting point of the mixture and mixed with the necessary quantity of water heated to substantially the same temperature and the resulting mixture is cooled to room temperature.

13. The use of the pearlescent concentrates claimed in any of claims 1 to 11 in a quantity of 0.5 to 10% by weight and, more particularly, 1.5 to 5% by weight for the production of clouded and pearlescent liquid aqueous preparations of water-soluble surfactants.

14. A process for the production of clouded and pearlescent liquid aqueous preparations of water-soluble surfactants, **characterized in that** the pearlescent concentrates claimed in any of claims 1 to 11 are added to the clear aqueous preparations in a quantity of 0.5 to 10% by weight and, more particularly, 1.5 to 5% by weight, based on the preparation, at a temperature of 0 to 40°C and are dispersed therein by stirring.

## Revendications

1. Concentré d'agent conférant un aspect nacré sous la forme d'une dispersion aqueuse avec de 10 à 40 % en poids de composants conférant un aspect nacré et de 15 à 55 % en poids d'émulsifiants,
**caractérisé en ce qu'**
il s'agit, pour les émulsifiants, d'alkylpolyglycosides de formule générale (I),
RO-(Z)ₓ (I),
dans laquelle R représente un radical alkyle comportant de 6 à 22 atomes de carbone, Z représente un mono- ou oligosaccharide, x représente un nombre allant de 1,1 à 5, ou de leurs produits d'addition avec de 1 à 10 molécules d'oxyde d'éthylène et/ou d'oxyde de propylène, et **en ce que** le concentré d'agent conférant un aspect nacré est dépourvu d'émulsifiants comportant des groupes -COO- et -OSO₃⁻.

2. Concentré de produit conférant un aspect nacré selon la revendication 1,
**caractérisé en ce que**
dans la formule (I) Z représente le glucose et x représente un nombre compris entre 1,1 et 2, en particulier un nombre compris entre 1,1 et 1,4.

3. Concentré de produit conférant un aspect nacré selon l'une des revendications 1 ou 2,
**caractérisé en ce qu'**
on y trouve comme composants conférant un aspect nacré
(A1) les esters de formule (II),
R¹-(OCₙH₂ₙ)ₓ-OR² (II)
dans laquelle R¹ représente un groupe acyle gras linéaire comportant de 14 à 22 atomes de carbone, R² est un hydrogène ou un groupe R¹, n = 2 ou 3 et x est un nombre allant de 1 à 4, et/ou
(A2) des monoalcanolamides de formule générale (III)
R³-CO-NH-X (III)
dans laquelle R³ est un groupe alkyle comportant de 8 à 22 atomes de carbone, en particulier de 8 à 18 atomes de carbone, et X représente un groupe -CH₂-CH₂-OH, un groupe -CH₂-CH₂-CH₂-OH ou un groupe -C-(CH₃)₂-OH, et/ou
(A3) des acides gras saturés linéaires comportant de 14 à 22 atomes de carbone, et/ou
(A4) les mono-, di- et triesters de glycérine avec des acides gras saturés linéaires comportant de 12 à 22 atomes de carbone.

4. Concentré de produit conférant un aspect nacré selon la revendication 3,
**caractérisé en ce que**
les composants formant un aspect nacré se composent à au moins 50 % en poids, en particulier à au moins 70 % en poids, de distéarate d'éthylène glycol.

5. Concentré de produit conférant un aspect nacré selon l'une des revendications 1 à 4,
**caractérisé en ce qu'**
il contient de 20 à 30 % en poids de composant formant un aspect nacré et de 15 à 40 % en poids d'émulsifiants.

6. Concentré de produits fournissant un aspect nacré selon l'une des revendications 1 à 5,
**caractérisé en ce qu'**
il contient en outre un alcool polyvalent à faible poids moléculaire.

7. Concentré de produit conférant un aspect nacré selon la revendication 6.
**caractérisé en ce que**
l'alcool polyvalent de faible poids moléculaire est contenu à des quantités allant de 0,1 à 15 % en poids.

8. Concentré de produits conférant un aspect nacré selon l'une des revendications 6 ou 7,
**caractérisé en ce que**
l'alcool polyvalente de faible poids moléculaire contient de 2 à 12 atomes de carbone et de 2 à 10 groupes hydroxyle.

9. Concentré de produit conférant un aspect nacré selon la revendication 8,
**caractérisé en ce que**
l'alcool polyvalent de faible poids moléculaire est choisi dans le groupe constitué par la glycérine, le 1,2-propylène glycol, le 1,3-propylène glycol, le glucose et le sorbitol.

10. Concentré de produit conférant un aspect nacré selon l'une des revendications 6 ou 7,
**caractérisé en ce que**
l'alcool polyvalent de faible poids moléculaire est choisi parmi les polyéthylène glycols, les polypropylène glycol et les polyglycérines ayant une masse molaire moyenne inférieure à 700 daltons.

11. Concentré de produit conférant un aspect nacré selon l'une des revendications 1 à 10,
**caractérisé en ce qu'**
Il contient en outre des émulsifiants non ionogènes à des quantités allant jusqu'à 50 % en poids, par rapport à la quantité d'alkylpolyglycoside.

12. Procédé de préparation de concentrés de produits conférant un aspect nacré selon l'une des revendications 1 à 11,
**caractérisé en ce qu'**
on mélange un mélange des composants à une température qui se situe de 1 à 30°C au-dessus du point de fusion du mélange, avec la quantité nécessaire d'eau, par exemple à la même température, puis on refroidit à la température ambiante.

13. Utilisation de concentrés de produits conférant un aspect nacré selon l'une des revendications 1 à 11 en une quantité de 0.5 à 10 % en poids, et de 1,5 à 5 % en poids, pour la préparation de compositions troubles et aqueuses liquides conférant un aspect nacré, de substances tensioactives solubles dans l'eau.

14. Procédé de préparation de préparations troubles et aqueuses liquides conférant un aspect nacré de matières tensioactives solubles dans l'eau,
**caractérisé en ce qu'**
on ajoute aux préparations aqueuses claires entre 0 et 40°C des concentrés de produits conférant un aspect nacré selon l'une des revendications 1 à 11, en une quantité allant de 0.5 à 10% en poids, en particulier de 1,5 à 5 % en poids, à la préparation et on les y répartit tout en agitant.
